(19) Europäisches Patentamt
European Patent Office
Office européen des brevets

(11) **EP 3 183 722 B1**

(12) **EUROPEAN PATENT SPECIFICATION**

(45) Date of publication and mention
of the grant of the patent:
**15.07.2020 Bulletin 2020/29**

(21) Application number: **15775798.0**

(22) Date of filing: **20.08.2015**

(51) Int Cl.:
**G08G 1/015** *(2006.01)*          **G08G 1/04** *(2006.01)*
**G08G 1/048** *(2006.01)*          **G01N 21/00** *(2006.01)*
**G01N 33/00** *(2006.01)*

(86) International application number:
**PCT/IB2015/056316**

(87) International publication number:
**WO 2016/027244 (25.02.2016 Gazette 2016/08)**

(54) **STATION FOR THE INTEGRATED MONITORING OF ENVIRONMENT AND TRAFFIC**

STATION FÜR DIE INTEGRIERTE ÜBERWACHUNG VON UMWELT UND VERKEHR

STATION INTEGREE DE SURVEILLANCE DE L'ENVIRONNEMENT ET TRAFIC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.08.2014 IT VI20140215**

(43) Date of publication of application:
**28.06.2017 Bulletin 2017/26**

(73) Proprietor: **Airtraff Di Mauro Zilio
36022 Cassola (VI) (IT)**

(72) Inventor: **ZILIO, Mauro
I-36022 Cassola (VI) (IT)**

(56) References cited:
**EP-A1- 1 521 226          WO-A1-2012/090235
WO-A2-03/017227          WO-A2-2005/077001
US-A1- 2008 024 323**

**Description**

Technical Field

The present invention finds its application in the field of the systems for monitoring vehicle traffics and in particular relates with a station for the integrated monitoring of environment and traffic.

State of the art

The current techniques for traffic monitoring provide in addition to the detection of parameters related to the volume of traffic, such as the flow of vehicles and noise, also the acquisition of environmental parameters such as, for example, the concentration of fine dust and/or other polluting elements.

The various acquired data are correlated with each other in order to obtain statistical data complete and reliable as much as possible in order to assess the trend of traffic in a given monitored zone in a given period of time or within the same day, so as to allow to take appropriate measures to ensure efficient management of traffic.

The same data may also be used for information purposes to alert motorists of the current traffic conditions.

From US2004/039517 it is known an integrated system for traffic monitoring that comprises a local unit designed to be positioned in correspondence of an area to be monitored. This unit is equipped both with sensors for detecting the traffic and with sensors for the detection of the noise and with sensors for the detection of pollutants in the air.

The data collected in this way are sent to a control center that will also receive additional information, such as weather data sent from a weather station, so that it can define a framework as complete as possible of the traffic conditions and then inform drivers. EP0527307 discloses, in turn, an integrated system for the detection of pollution from traffic which includes fixed and mobile units for detecting parameters related to environmental pollution for the acquisition of local data to be sent to a central control. The latter will also get weather data, in particular short-term forecasts sent from a weather station, so it can provide forecasts on pollution.

However, these known solutions are affected by several drawbacks, a first of which is constituted by the fact that none of the systems described above provides a complete and actual outline of the environmental and traffic conditions of the monitored area.

In particular, with regard to the weather conditions, the above cited solutions detect conditions that are of general type and not local, i.e. related to areas wider than those monitored, possibly based on forecasts. As a consequence the weather conditions in the region that includes the monitored area are not always uniform throughout the region and therefore not reflect the actual situation in this area.

WO 2012/090235 discloses a system for monitoring the environment which provides for the detection of data relating to the traffic in an area monitored by placing suitable audio sensors adapted to detect data relating to the type, direction and speed of the passing vehicles and further sensors adapted to records data related to environmental pollution. The same audio sensors used for the detection of vehicular traffic can also be used to obtain some information about the weather condition, in particular to the possible presence of rain, through the insulation of the specific noise.

It seems evident that this system is unable to provide a comprehensive picture of the weather situation in the monitored area.

Moreover, none of the known solutions described above is adapted to interpolate the various data in order to obtain reliable evaluations on traffic conditions and of the pollution linked thereto and in particular to eliminate any detection anomalies due to the presence of phenomena that can alter the other types of collected data.

Not least, the systems described above are complex systems consisting of several units located in different areas from each other and none of which appears to be suited to perform all the measurements in a completely autonomous way.

Scope of the invention

The object of the present invention is to overcome the above mentioned drawbacks, by providing a station for integrated monitoring of environment and traffic that has high efficiency and relative cheapness.

A particular object is to provide a station for integrated monitoring of environment and traffic that is capable of acquiring the actual environmental data in real time of an area to be monitored both as regards the flows of traffic that regarding the concentrations of the pollutants and weather conditions.

A particular object is to provide a station for integrated monitoring of environment and traffic that is capable of integrating the data thus collected in order to provide extremely reliable information on the environmental and traffic conditions and achieve reliable statistics.

Still another object is to provide a station for integrated monitoring of environment and traffic that is completely autonomous and possibly transportable.

Still another object is to provide a station for integrated monitoring of environment and traffic with relatively reduced dimensions adapted to be easily positioned and used where it is not possible to locate the conventional stations, allowing the capillary monitoring of the territory. A further object of the present invention is to provide a station for integrated monitoring of environment and traffic that can be integrated into systems for detecting the air quality in order to provide information relative to the pollution produced by vehicular traffic.

Not last object of the present invention is to provide a station for integrated monitoring of environment and traffic that ensures high precision in traffic monitoring, even in conditions of high traffic flow.

These objects, as well as others which will appear more clearly hereinafter, are achieved by a station for integrated monitoring of environment and traffic that, according to claim 1, comprises a local unit designed to be positioned in correspondence of an area to be monitored, said local unit comprising first means for collecting first data related to the concentration of pollutants in the air, second means for monitoring the flow of vehicles in the monitored area and for collecting corresponding second data, a data processing unit adapted to receive said first and said second data for their correlation and the production of information related to traffic and the air quality for the monitored area in a predetermined period of time.

The local unit further comprises third means for collecting third data relating to weather conditions in the monitored area adapted to be sent to said data processing unit.

Thanks to this combination of features the station will allow to know all the actual environmental and traffic conditions in the monitored area in real time and with relatively high precision, therefore based on real data and not on forecasts.

Furthermore, all the collected data will relates to environmental parameters closely related to the specific monitored area.

For example, said third data collecting means may comprise a weather module associated with said local unit and provided with one or more sensors for real-time detection of meteorological parameters of the monitored area selected from the group comprising temperature, wind, humidity, barometric pressure, rain and the like.

Suitably, said central processing unit may be adapted to correlate said first and said second data with said third data to allow the validation of the first and/or second data. In particular, the first data may be validated by the control unit only in the moment in which one or more of said third said data is within a predetermined range of values.

This will make it possible to discard the traffic and/or pollution data detected at values weather falling within predetermined ranges that would make the first and/or second data not reliable.

A typical example is the one in which the monitored area is affected by especially intense rain or wind or by particularly high temperatures, so that the values relative to the measurements of concentrations of pollutants in the air may not reflect actual traffic flow.

Preferably, the first data collecting means may comprise a base module for the detection of gaseous substances and having one or more sensors selected from the group comprising CO, 03, C6H6, NOx sensors and the like.

Moreover, the first collecting means may also comprise a secondary module connected to said base module for the detection of polluting powders and having one or more sensors selected from the group comprising PM10, PM1, PM2.5 sensors and the like.

This will make it possible to have a complete screening of the concentrations of the main pollutants typical of a traffic flow.

Advantageously, the second collecting data means may include a traffic module adapted to acquire information concerning the presence and/or passage of vehicles transiting in the monitored area and to generate second data relative to the volume of traffic, the type of vehicles and the speed average thereof.

Preferably, said traffic module may comprise one or more HD sensors radar, which will allow to monitor the traffic flow with great precision, allowing to discriminate with low margin of error some features of the flow of vehicles such as the number, type and average speed of vehicles, distances between the vehicles and also their directions of travel.

The second data collecting means may also incorporate one or more video devices adapted to detect and record images in real time of the traffic flowing through in the monitored area, and optionally a phonometric module at least of grade 1 provided with an adjustable microphone and adapted to perform analysis of the sound pressure.

Advantageously, the local unit may have a total weight less than 750Kg and to comprise a transportable cart, so as to be joined to a towing hook of a car or other vehicle for whose driving is sufficient a common driving license, making the whole station easily deployable. Suitably, said local unit may comprise means for autonomous power supply of said first, second and third means even in the absence of connection to the electricity network, which means may be associated with a containment shell of the carriage.

For example, the power supply means may comprise one or more photovoltaic panels defining or associated to the roof and/or to a side wall of said shell.

In addition or alternatively, the power supply means may comprise further electrical power supply devices from renewable sources, such as microwind blades.

In this way, the station will be completely autonomous and self-standing to be easily transported and positioned at any point, guaranteeing high flexibility of use since it does not require connection to the electricity network and being able to also be integrated into systems for detecting the quality of air to provide an assessment of the pollution produced by vehicular traffic.

The invention is defined in the appended claim 1. Advantageous embodiments of the invention are obtained according to the dependent claims.

## Brief description of the drawings

Further features and advantages of the invention will become more apparent in light of the detailed description of a preferred but not exclusive embodiment of a station for integrated monitoring of environment and traffic, illustrated by way of non-limiting example with the aid of the accompanying drawing, wherein:

FIG. 1 is a first perspective view of a station according to the invention;
FIG. 2 is a second perspective view of a station according to the invention;
FIG. 3 is a block-diagram schematically showing the structure of a station according the invention;
FIG. 4 shows a graph relating to a measurement performed by a station according to the invention.

## Best mode of carrying out the invention

With reference to the accompanying figures, it is shown a station for integrated monitoring of environment and traffic intended to be arranged close to urban or extra-urban roads, including motorways, and in any case in correspondence of areas affected by vehicular traffic, in order to monitor in real time traffic flows and to measure the environmental pollution and possibly acoustic pollution product instantly from said flow.

The monitoring station may advantageously be integrated within a network constituted by a plurality of identical stations or the like connected to a central remote management that will be used to receive the data and to interpret the subsequent management of traffic and information to related thereto, according to known methods.

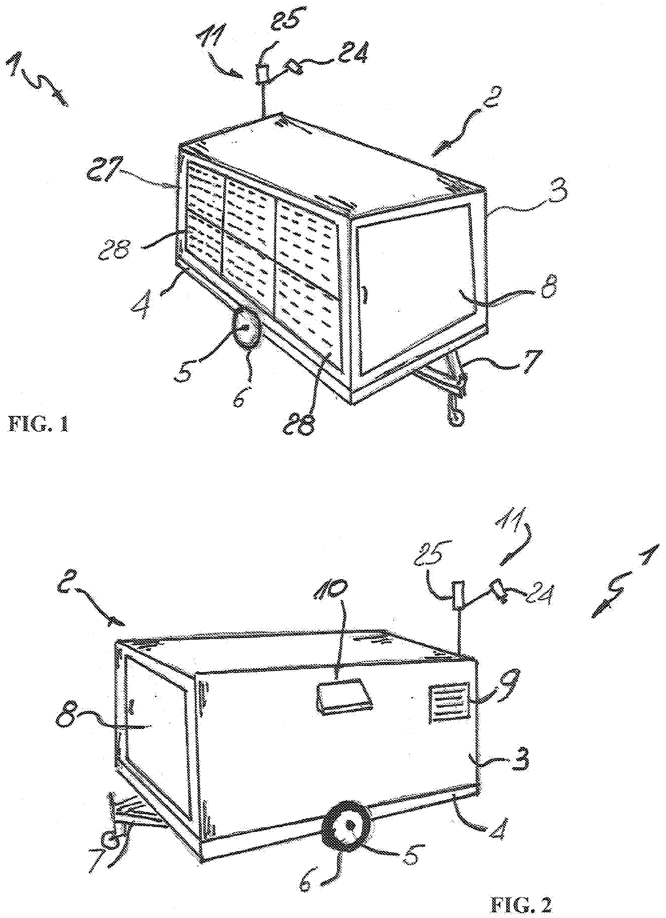

**Fig. 1** shows a particular configuration of the station according to the invention, generally indicated by **1**, having a particularly compact conformation to be easily transported and placed even in relatively restricted spaces.

The monitoring station **1** will be configured to comply with the guidelines laid down in the Italian Legislative Decree no. 155/2010 and thus also implement the provisions of Community law No. 50/2008.

In particular, the station **1** comprise a local unit **2** designed to be positioned in correspondence of the area to be monitored.

In its preferred embodiment, not limiting the scope of protection of the present invention, the local unit **2** as a whole will have relatively low weight and preferably not more than 750Kg with maximum dimensions falling within the limits for light trailers, so as to be transported by a motor vehicle.

The local unit **2** will comprise a box-like containment shell **3** placed on a lower frame **4** having an axle **5** for a pair of wheels **6**, and optionally provided with a tow hook **7**.

The containment shell **3** will also be equipped with a tailgate **8** to access an inner compartment, wherein the electrical and electronic components necessary for the measurements will be housed, and with ventilation grids **9**, visible in **Fig. 2**.

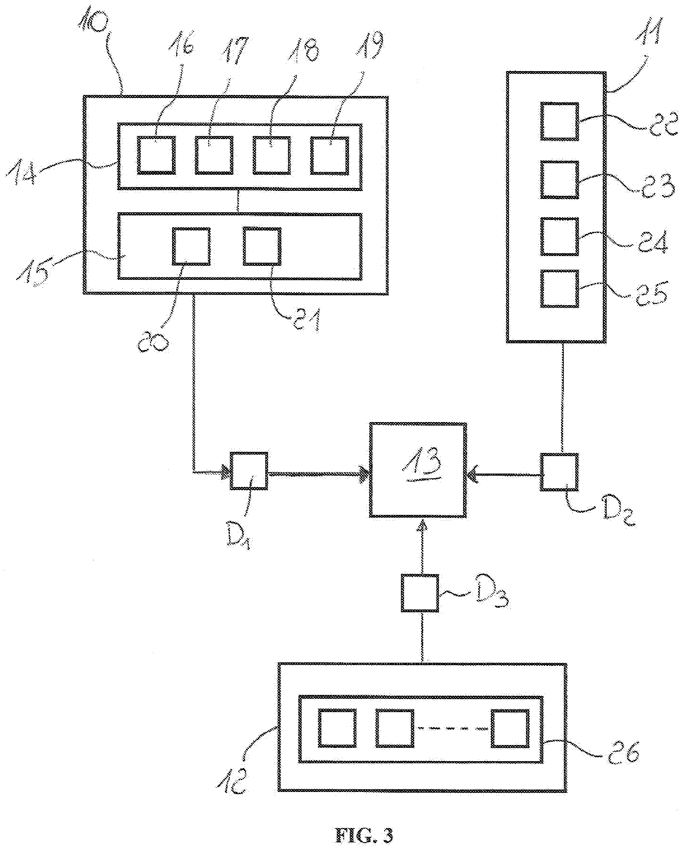

In **Fig. 3** is instead shown in a schematic way the configuration of the electrical and electronic components associated to the local unit **2**.

In particular, the local unit **2** comprise first means **10** for collecting first data **D1** related to the concentration of pollutants in the air, second means **11** for monitoring the flow of vehicles in the monitored area and the collection of corresponding second data **D2** and third means **12** for collecting of third data **D3** relating to the meteorological conditions in the monitored area. The first, second and third data **D1**, **D2**, **D3** will be designed to be sent to a data processing unit **13** located into the internal compartment of the local unit **2** and which in turn will be connected to the remote unit, not shown.

The data processing unit **13** will preferably be provided with an its own data logger for recording data, and is adapted to correlate the first and second data **D1**, **D2** for the production of information concerning the traffic and the air quality for the monitored area over a predetermined time period.

At the same time, the data processing unit **13** will be adapted to correlate the first and second data **D1**, **D2** with the third data **D3** for the validation of the first data **D1** and/or the second data **D2**.

In particular, the first data **D1** and/or the second data **D2** may be considered reliable, and therefore validated in the moment in which one or more of the third data **D3**, preferably all, have a value falling within a predetermined range of values.

The first data collecting means **10** will comprise a base module **14** for the detection of gaseous pollutants and a secondary module **15** wired to the base module **14**.

The base module **14** will comprise a plurality of sensors **16**, **17**, **18**, **19** for the detection of pollutants such as CO, 03, C6H6, NOx. **Table 1** below shows a non-limiting example of the operational features of the sensors **16÷19** of the base module **14**.

**TABLE 1**

| Sensor | Value Range | Precision | Resolution | Accuracy |
|---|---|---|---|---|
| CO | 0-100 mg/m3<br>0-80ppm | < 2% | 0,1mg/m3 | ± 0,5mg/m3 |
| 03 | 20-500μg/m3<br>10-200 ppb | < 10% | 1,0μg/m3 | ± 10 μg/m3 |
| C6H6 | 0-100μg/m3<br>0-30ppb | < 2% | 0,1 μg/m3 | ± 1,0 mg/m3 |
| Nox | 0-800μg/m3<br>0-500ppb | < 2% | 0,1 μg/m3 | ± 10 μg/m3 |

The secondary module **15** will comprise sensors **20**, **21** for detecting fine particles such as PM10, PM2.5 and PM1 sensors.

**Table 2** shows a non-limiting example of the technical specifications for the sensors **20**, **21** of the secondary module **15**.

**TABLE 2**

| Sensor | Value Range | Precision | Resolution | Accuracy |
|---|---|---|---|---|
| PM10 | 0,001-100mg/m3 | < 2% | 0,001mg/m3<br>oppure 0,1% | ± 5% |
| PM2,5 | 0,001-100mg/m3 | < 2% | 0,001mg/m3<br>oppure 0,1% | ± 5% |
| PM1 | 0,001-100mg/m3 | < 2% | 0,001mg/m3<br>oppure 0,1% | ± 5% |

In a exemplificative configuration, preferred but not exclusive for the invention, the secondary module **15** will be adapted to detect fine particles with a Nephelometric method and the related data will be available continuously with a minimum interval of two seconds to be stored and transmitted by modem.

The second data collecting means **11** will comprise a traffic module **22** adapted to acquire information relative to the presence of vehicles passing in the monitored area and to generate second data **D2** relating to the volume of traffic, to the type of vehicles and to the average speed thereof.

For example, the traffic module **22** will include a plurality of HD radar sensors **23** that in an example of embodiment will be of the double channel type for precision monitoring of the behavior of the vehicles and to guarantee functionality even with adverse weather conditions. The radar **22** will not necessarily be inserted inside the local unit **2** but may be located at certain points of the area to be monitored according to the needs.

The radar **22** may have various features, and for example may be adapted to simultaneously manage a relatively large number of lanes and vehicle classes, for example 12 lanes, or even more, and 8 classes of vehicles, to count the vehicles, to measure the average speed, to calculate the relative distances and to discriminate the direction of travel.

Conveniently, the radar **22** will be configured to collect data with extremely high accuracy, being able to discriminate the volume of traffic with a maximum precision up to 98% and by 90% for each lane, with a maximum precision up to 90% in the classification of the vehicles and with maximum error of 5% with regard to the measures of the average speed.

The collected data will be sent in real time via the web to the remote management unit.

The second data collecting means **11** may also comprise one or more video devices **24** adapted to detect and record images in real time of the traffic flowing through in the monitored area.

In this way, in the case of second data **D2** with anomalous detection values it will be possible to immediately verify if particular events have occurred that could have caused this anomaly. By way of example, in case of an abnormal increase of the data relating to fine dust or other products of fuel combustion, it is possible to check the possible presence of motor vehicles running close to the local unit **2**.

In particular, the local unit **2** may be provided with a HD video camera for the acquisition and possible recording of video images.

By way of example, the camera may be equipped with video sensor of 3 megapixels, night video sensors, local storage recording, streaming and real-time video management, alarms with function of motion detection, infrared sensor.

The second data collecting means **11** may optionally be also provided with a phonometric module **25** at least of class 1 with orientable microphone adapted to perform analysis of the sound pressure, also by means of frequency analysis.

The third data collecting means **12** will comprise a weather module **26** also located directly on the local unit **2** and provided with one or more sensors for real-time detection of meteorological parameters of the monitored area, such as temperature, wind, humidity, pressure barometric, rain and the like.

The local unit **2** will also be provided with means **27** for autonomous power supply of the various electrical and electronic components, including the first, second and third means, even in the absence of connection to the electricity network.

In particular, the electrical power supply means **27** will comprise one or more photovoltaic panels **28**, for example six panels, positioned directly on the shell **3**, for example on the roof or on one or more outer side walls.

According to a not shown configuration, in addition or as an alterative to the panels **28**, the local unit **2** may be provided with additional power supply devices from renewable sources, such as microwind blades.

The electric power supply means **27** may also comprise a buffer battery, not shown, to be used in all those cases in which no other power source is available.

Preferably, the battery may be used continuously for the power supply of the entire station and will be connected to the panels **28** and/or other power device from renewable sources that have the task to recharge it and ensure sufficient charge during night.

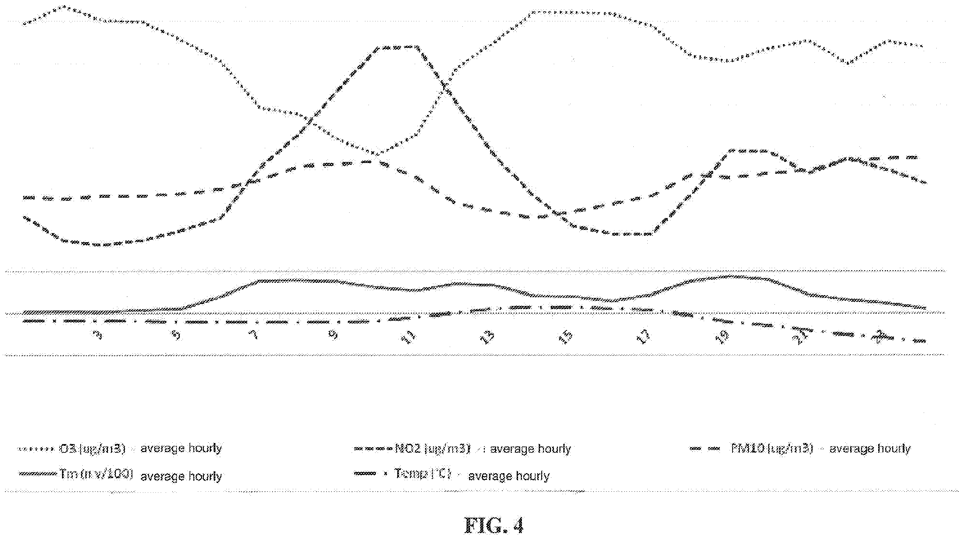

**Fig. 4** shows a exemplificative graph of a monitoring in a time span of 24 hours and in which the detected first data **D1** are related to the average concentrations in a subperiod **p** of an hour of the detected values of ozone 03, nitrogen dioxide NO2 and PM10 particulate. These data **D1** are correlated to second data **D2** relating to the traffic average **Tm**, measured as a function of the average speed v of the vehicle according to the formula

$$Tm = n * v / 100$$

In addition, both the first data **D1** and the second data **D2** are correlated to third data **D3** relating to weather conditions, and that in the case of the example are represented by the average temperature **T** detected in each subperiod **p**.

The graph of **Fig. 4** was obtained using the data shown in **Table 3** below.

**TABLE 3**

| P | 03 (ug/m3) average hourly | NO2 (ug/m3) average hourly | PM10 (ug/m3) average hourly | Tm (n v/100) average hourly | Temp (°C) average hourly |
|---|---|---|---|---|---|
| 1 | 69,3 | 23,2 | 27,9 | 0,4 | -1,62 |
| 2 | 73,4 | 17,6 | 27,5 | 0,6 | -1,4 |
| 3 | 70,1 | 1,64E+01 | 28,2 | 0,4 | -1.60E+00 |
| 4 | 6,97E+01 | 17,5 | 28,2 | 0,98 | -1,7 |
| 5 | 65,4 | 19,9 | 28,7 | 1,3 | -1,84E+00 |
| 6 | 60,3 | 2,28E+01 | 29,7 | 4,2 | -1,74E+00 |
| 7 | 49,3 | 34,7 | 31,8 | 7,8 | -2.02E+00 |
| 8 | 47,7 | 43 | 35,2 | 8,1 | -1,98 |
| 9 | 4,16E+01 | 53,6 | 35,8 | 7,7 | -1,86 |
| 10 | 3,80E+01 | 6,33E+01 | 36,4 | 6,3 | -1,62 |
| 11 | 42,8 | 63,7 | 32,5 | 5,5 | -0,64 |
| 12 | 58,6 | 50,4 | 26,3 | 7,3 | 0,28 |
| 13 | 65,1 | 37,5 | 24,4 | 6,8 | 1,22 |

(continued)

| P | 03 (ug/m3) average hourly | NO2 (ug/m3) average hourly | PM10 (ug/m3) average hourly | Tm (n v/100) average hourly | Temp (°C) average hourly |
|---|---|---|---|---|---|
| 14 | 72 | 28 | 22,9 | 4,3 | 1,62 |
| 15 | 71,9 | 21 | 24,4 | 4,1 | 1,52 |
| 16 | 71,5 | 19,1 | 26,2 | 3,1 | 1,32 |
| 17 | 68,6 | 19,1 | 28,1 | 4,7 | 0,94 |
| 18 | 6,14E+01 | 28,5 | 33,1 | 7,9 | -0,4 |
| 19 | 60,2 | 39 | 32,5 | 9 | -1,98E+00 |
| 20 | 63,3 | 38,7 | 33,5 | 8,2 | -2,78 |
| 21 | 65,2 | 33,5 | 34,3 | 4,7 | -3,96 |
| 22 | 59,6 | 37,1 | 36,5 | 3,3 | -4,98 |
| 23 | 65,1 | 34,4 | 37 | 2,6 | -5,66 |
| 24 | 63,6 | 31,1 | 37,4 | 1,2 | -6,58 |

From the graph it is first possible to observe that for the entire monitoring period the average temperature **T** stays on substantially constant and relatively low values, so that it is possible to consider valid the measurements relating to the first data **D1**.

In addition, it is also possible to observe the opposite trend curves related to 03 and NO2, with the increase of the latter during the hottest hours in correspondence of the decrease of 03, in a manner consistent with the trend of ozone 03 to turn into NO2 as the irradiation increases.

The comparison between the two curves thus allows to obtain a further validation of collected data.

Similarly the substantially symmetrical trend between the curve of the average traffic **Tm** and that of the PM10 also represents an index relating to the correctness of the monitoring and the absence of anomalies.

From above it appears evident that the station according to the invention reaches the intended objects.

The station according to the invention is susceptible of numerous modifications and variations, all falling within the inventive concept expressed in the accompanying claims. All the details may be replaced with other technically equivalent elements, and the materials may be different according to requirements, without departing from the scope of the present invention.

Even if the station has been described with particular reference to the attached figures, reference numbers used in the description and in the claims are used to improve the intelligence of the invention and do not constitute any limitation the claimed scope.

**Claims**

1. A station for the integrated monitoring of environment and traffic, comprising a local unit (**2**) adapted to be positioned at an area to be monitored, said local unit (**2**) comprising:

   - first means (**10**) for collecting first data (**D1**) relative to the concentration of pollutants in the air;
   - second means (**11**) for monitoring the traffic flow in the monitored area and for collecting corresponding second data (**D2**);
   - a data processing unit (**13**) adapted to receive said first data (**D1**) and said second data (**D2**) for the correlation thereof and for generating information relative to the traffic and to the air quality in the monitored area in a predetermined time period;

   **characterized in that** said local unit (**2**) comprises third means (**12**) for collecting third data (**D3**) relative to the weather condition in the monitored area adapted to be transmitted to said data processing unit (**13**), said third data (**D3**) relating to at least one between amount of rain, wind intensity, atmospheric pressure and being measured in the monitored area, said data processing unit (**13**) being adapted to correlate said first data (**D1**) and said second data (D2) to said third data (**D3**) for validating said first data (**D1**) and said second data (D2) when said third data (**D3**) are inside a respective predetermined range of values.

2. Station as claimed in any preceding claim, **characterized in that** said first data collection means (**10**) comprise a base module (**14**) for detecting gaseous substances, which base module (**14**) has one or more sensors (**16**, **17**, **18**, **19**) selected into the group comprising CO, O3, C6H6, NOx sensors.

3. Station as claimed in claim 2, **characterized in that** said first data collection means (**10**) comprise a secondary module (**15**) connected to said base module (**14**) for detecting polluting dusts and having one or more sensors (**20**, **21**) selected in the group comprising PM10, PM2,5, PM1 sensors.

4. Station as claimed in any preceding claim, **characterized in that** said second data collection means (**11**) comprise a traffic module (**22**) adapted to collect information relative to the presence of vehicles circulating through the monitored area and to generate second data (**D2**) relative to the traffic flow, to the types of the vehicles and to the average speed thereof.

5. Station as claimed in claim 4 **characterized in that** said traffic module (**22**) comprises one or more HD radar sensors (**23**).

6. Station as claimed in claim 5 **characterized in that** said second data collection means (**11**) comprise one or more video devices (**24**) adapted to detect and record in real time imagines of the traffic passing through in the monitored area.

7. Station as claimed in claim 6, **characterized in that** said second data collection means (**11**) comprise a phonometric module (**25**) at least of class 1 provided with an adjustable microphone adapted to execute sound pressure analysis.

8. Station as claimed in any preceding claim, **characterized in that** said third data collection means (**12**) comprise a weather module (**26**) associated with said local unit (**2**) and provided with one or more sensors for detecting in real time weather parameters in the monitored area, which parameters being selected into the group comprising temperature, wind intensity, moisture, barometric pressure and/or amount of rain.

9. Station as claimed in any preceding claim, **characterized in that** said local unit (**2**) comprises autonomous electric power means (**27**) for powering said first means (**10**), said second means (**11**) and said third means (**12**).

10. Station as claimed in claim 9 **characterized in that** said autonomous electric power means (**27**) comprise one or more photovoltaic panels (**28**) and/or further power devices from renewable power sources.

11. Station as claimed in any preceding claim, **characterized in that** said local unit (**2**) has a total weight lower than 750Kg and comprises a transportable cart (**7**) with a containment shell (**3**) adapted to contain at least said processing unit (**13**).

12. Station as claimed in claim 11 **characterized in that** said base module (**14**), said secondary module (**15**) and said weather module (**26**) are firmly associated with said shell (**2**).

13. Station as claimed in claim 10 and 11 **characterized in that** said electric power means (**27**) comprise one or more photovoltaic panels (**28**) defining or associated with the roof and/or with at least one side wall of said shell (**3**).

**Patentansprüche**

1. Station zur integrierten Überwachung von Umgebung und Verkehr, umfassend eine lokale Einheit (**2**), die angepasst ist, um in einem zu überwachenden Bereich positioniert zu werden, wobei die lokale Einheit (**2**) umfasst:

- erste Mittel (**10**) zur Erfassung der ersten Daten (**D1**) in Bezug auf die Schadstoffkonzentration in der Luft;
- zweite Mittel (**11**) zum Überwachen des Verkehrsflusses in dem überwachten Bereich und zum Sammeln entsprechender zweiter Daten (**D2**);
- eine Datenverarbeitungseinheit (**13**), die angepasst ist, um die ersten Daten (**D1**) und die zweiten Daten (**D2**) zu empfangen, um sie zu korrelieren und Informationen in Bezug auf den Verkehr und die Luftqualität in dem überwachten Bereich in einem vorbestimmten Zeitraum zu erzeugen;

**dadurch gekennzeichnet, dass** die lokale Einheit (**2**) ein drittes Mittel (**12**) zum Sammeln von dritten Daten (**D3**) in Bezug auf die Wetterbedingungen in dem überwachten Bereich umfasst, die zur Übertragung an die Datenverarbeitungseinheit (**13**) angepasst sind, wobei die dritten Daten (**D3**) in Bezug auf mindestens eine zwischen Regenmenge, Windintensität, atmosphärischem Druck und Messung in dem überwachten Bereich, wobei die Datenverarbeitungseinheit (**13**) angepasst ist, um die ersten Daten (**D1**) und die zweiten Daten (**D2**) mit den dritten zu korrelieren Daten (**D3**) zum Validieren der ersten Daten (**D1**) und der zweiten Daten (**D2**), wenn die dritten Daten (**D3**) innerhalb eines jeweils vorbestimmten Wertebereichs liegen.

2. Station, wie in einem vorhergehenden Anspruch beansprucht, **dadurch gekennzeichnet, dass** die erste Datenerfassungseinrichtung (**10**) ein Basismodul (**14**) zum Erfassen gasförmiger Substanzen umfasst, wobei das Basismodul (**14**) einen oder mehrere Sensoren (**16**, **17**, **18**, **19**) aufweist ausgewählt in die Gruppe umfassend CO-, O3-, C6H6-, NOx-Sensoren.

3. Station nach Anspruch 2, **dadurch gekennzeichnet, dass** die erste Datenerfassungseinrichtung (**10**) ein Sekundärmodul (**15**) umfasst, das mit dem Basismodul (**14**) verbunden ist, um umweltschädliche Stäube zu erfassen und einen oder mehrere Sensoren (**20**, **21**) aufweist ausgewählt in der Gruppe bestehend aus PM10-, PM2,5-, PM] -Sensoren.

4. Station, wie in einem vorhergehenden Anspruch beansprucht, **dadurch gekennzeichnet, dass** die zweite Datenerfassungseinrichtung (**11**) ein Verkehrsmodul (**22**) umfasst, das dazu ausgelegt ist, Informationen bezüglich des Vorhandenseins von Fahrzeugen, die durch den überwachten Bereich zirkulieren, zu sammeln und zweite Daten (**D2**) zu erzeugen im Verhältnis zum Verkehrsfluss, zu den Fahrzeugtypen und zu deren Durchschnittsgeschwindigkeit.

5. Station nach Anspruch 4, **dadurch gekennzeichnet, dass** das Verkehrsmodul (**22**) einen oder mehrere HD-Radarsensoren (**23**) umfasst.

6. Station nach Anspruch 5, **dadurch gekennzeichnet, dass** die zweite Datenerfassungseinrichtung (**11**) ein oder mehrere Videogeräte (**24**) umfasst, die dazu ausgelegt sind, in Echtzeit Vorstellungen des in dem überwachten Bereich durchlaufenden Verkehrs zu erfassen und aufzuzeichnen.

7. Station nach Anspruch 6, **dadurch gekennzeichnet, dass** die zweite Datenerfassungseinrichtung (**11**) ein phonometrisches Modul (**25**) mindestens der Klasse 1 umfasst, das mit einem einstellbaren Mikrofon versehen ist, das zur Durchführung einer Schalldruckanalyse ausgelegt ist.

8. Station, wie in einem vorhergehenden Anspruch beansprucht, **dadurch gekennzeichnet, dass** die dritte Datenerfassungseinrichtung (**12**) ein Wettermodul (**26**) umfasst, das der lokalen Einheit (**2**) zugeordnet ist und mit einem oder mehreren Sensoren zum Erfassen von Wetterparametern in Echtzeit versehen ist im überwachten Bereich, welche Parameter in die Gruppe ausgewählt werden, die Temperatur, Windintensität, Feuchtigkeit, Luftdruck und/oder Regenmenge umfasst.

9. Station, wie in einem vorhergehenden Anspruch beansprucht, **gekennzeichnet dadurch, dass** die lokale Einheit (**2**) autonome elektrische Energieeinrichtung (**27**) zum Versorgen der ersten Mittel (**10**), der zweiten Mittel (**11**) und der dritten Mittel (**12**) umfasst.

10. Station nach Anspruch 9, **dadurch gekennzeichnet, dass** das autonome Stromversorgungsmittel (**27**) ein oder mehrere Photovoltaikmodule (**28**) und/oder weitere Stromversorgungsvorrichtungen aus erneuerbaren Energiequellen umfasst.

11. Station, wie in einem vorhergehenden Anspruch beansprucht, **dadurch gekennzeichnet, dass** die örtliche Einheit (**2**) ein Gesamtgewicht von weniger als 750 kg aufweist und einen transportablen Wagen (**7**) mit einer Rückhaltehülle (**3**) umfasst, die so ausgelegt ist, dass sie mindestens die Verarbeitungseinheit (**13**).

12. Station nach Anspruch 11, **dadurch gekennzeichnet, dass** das Basismodul (**14**), das Sekundärmodul (**15**) und das Wettermodul (**26**) fest mit der Hülle (**2**) verbunden sind.

13. Station nach Anspruch 10 und 11, **dadurch gekennzeichnet, dass** die elektrischen Leistungsmittel (**27**) ein oder mehrere Photovoltaikmodule (**28**) umfassen, die das Dach und/oder mindestens eine Seitenwand der Hülle (**3**) definieren oder mit diesem verbunden sind.

**Revendications**

1. Station de surveillance intégrée de l'environnement et du trafic, comprenant une unité locale (**2**) adaptée pour être positionnée dans une zone à surveiller, ladite unité locale (**2**) comprenant:

   - des premiers moyens (**10**) pour collecter des premières données (**D1**) relatives à la concentration de polluants dans l'air;
   - des seconds moyens (**11**) pour surveiller le flux de trafic dans la zone surveillée et pour collecter les secondes données correspondantes (**D2**);
   - une unité de traitement de données (**13**) adaptée pour recevoir lesdites premières données (**D1**) et lesdites secondes données (**D2**) pour leur corrélation et pour générer des informations relatives au trafic et à la qualité de l'air dans la zone surveillée dans une période de temps prédéterminée ;

   **caractérisé en ce que** ladite unité locale (**2**) comprend des troisièmes moyens (**12**) pour collecter des troisièmes données (**D3**) relatives aux conditions météorologiques dans la zone surveillée adaptées pour être transmises à ladite unité de traitement de données (**13**), lesdites troisièmes données (**D3**) concernant au moins une quantité de pluie, l'intensité du vent, la pression atmosphérique et étant mesurée dans la zone surveillée, ladite unité de traitement de données (**13**) étant adaptée pour corréler lesdites premières données (**D1**) et lesdites secondes données (**D2**) avec lesdites troisièmes des données (**D3**) pour valider lesdites premières données (**D1**) et lesdites secondes données (**D2**) lorsque lesdites troisièmes données (**D3**) se trouvent à l'intérieur d'une plage de valeurs prédéterminée respective.

2. Station selon l'une quelconque des revendications précédentes, **caractérisée en ce que** lesdits premiers moyens de collecte de données (**10**) comprennent un module de base (**14**) pour détecter des substances gazeuses, lequel module de base (**14**) comporte un ou plusieurs capteurs (**16**, **17**, **18**, **19**) sélectionnés dans le groupe comprenant les capteurs CO, O3, C6H6, NOx.

3. Station selon la revendication 2, **caractérisée en ce que** lesdits premiers moyens de collecte de données (**10**) comprennent un module secondaire (**15**) connecté audit module de base (**14**) pour détecter les poussières polluantes et comportant un ou plusieurs capteurs (**20**, **21**) sélectionnés dans le groupe comprenant les capteurs PM10, PM2,5, PM1.

4. Station selon l'une quelconque des revendications précédentes, **caractérisée en ce que** lesdits seconds moyens de collecte de données (**11**) comprennent un module de circulation (**22**) adapté pour collecter des informations relatives à la présence de véhicules circulant dans la zone surveillée et pour générer des secondes données (**D2**) par rapport à la circulation, aux types de véhicules et à leur vitesse moyenne.

5. Station selon la revendication 4, **caractérisée en ce que** ledit module de trafic (**22**) comprend un ou plusieurs capteurs radar HD (**23**).

6. Station selon la revendication 5, **caractérisée en ce que** lesdits seconds moyens de collecte de données (**11**) comprennent un ou plusieurs dispositifs vidéo (**24**) adaptés pour détecter et enregistrer des images en temps réel du trafic traversant dans la zone surveillée.

7. Station selon la revendication 6, **caractérisée en ce que** lesdits seconds moyens de collecte de données (**11**) comprennent un module phonométrique (**25**) au moins de classe 1 muni d'un microphone réglable apte à exécuter une analyse de pression acoustique.

8. Station selon l'une quelconque des revendications précédentes, **caractérisée en ce que** lesdits troisièmes moyens de collecte de données (**12**) comprennent un module météo (**26**) associé à ladite unité locale (**2**) et pourvu d'un ou plusieurs capteurs pour détecter en temps réel des paramètres météo dans la zone surveillée, quels paramètres sont sélectionnés dans le groupe comprenant la température, l'intensité du vent, l'humidité, la pression barométrique et/ou la quantité de pluie.

9. Station selon l'une quelconque des revendications précédentes, **caractérisée en ce que** ladite unité locale (**2**) comprend des moyens autonomes d'alimentation électrique (**27**) pour alimenter lesdits premiers moyens (**10**), lesdits seconds moyens (**11**) et lesdits troisièmes moyens (**12**).

10. Station selon la revendication 9, **caractérisée en ce que** lesdits moyens autonomes d'alimentation électrique (**27**) comprennent un ou plusieurs panneaux photovoltaïques (**28**) et/ou d'autres dispositifs d'alimentation à partir de sources d'énergie renouvelables.

11. Station selon l'une quelconque des revendications précédentes, **caractérisée en ce que** ladite unité locale (**2**) a un poids total inférieur à 750 kg et comprend un chariot transportable (**7**) avec une coque de confinement (**3**) apte à contenir au moins ladite unité de traitement (**13**).

12. Station selon la revendication 11, **caractérisée en ce que** ledit module de base (**14**), ledit module secondaire (**15**) et ledit module météo (**26**) sont solidement associés à ladite coque (**2**).

13. Station selon les revendications 10 et 11, **caractérisée en ce que** lesdits moyens d'alimentation électrique (**27**) comprennent un ou plusieurs panneaux photovoltaïques (**28**) définissant ou associés au toit et/ou à au moins une paroi latérale de ladite coque (**3**).

EP 3 183 722 B1

FIG. 1

FIG. 2

FIG. 3

O3 (ug/m3) - average hourly      NO2 (ug/m3) - average hourly      PM10 (ug/m3) - average hourly

Tm (m v/100) - average hourly      Temp (°C) - average hourly

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004039517 A
- EP 0527307 A

- WO 2012090235 A